# EUROPÄISCHE PATENTSCHRIFT

(11) **EP 2 598 241 B1**
(45) Veröffentlichungstag und Bekanntmachung des Hinweises auf die Patenterteilung: **14.05.2014**
(21) Anmeldenummer: 11741171.0
(22) Anmeldetag: 26.07.2011
(51) Int. Cl.: B01J 23/96, B01J 38/20, C07C 209/36

(54) **VERFAHREN ZUR WIEDERHERSTELLUNG VON KATALYSATORAKTIVITÄT**
PROCESS FOR RESTORING CATALYST ACTIVITY
PROCÉDÉ DE RESTAURATION DE L'ACTIVITÉ D'UN CATALYSEUR

(30) Priorität: 30.07.2010 DE 102010038680
(43) Veröffentlichungstag der Anmeldung: 05.06.2013
(73) Patentinhaber: Bayer Intellectual Property GmbH, 40789 Monheim (DE)
(72) Erfinder: MERKEL, Michael, 40223 Düsseldorf (DE); KNAUF, Thomas, 41542 Dormagen (DE); WILKE, Karl-Heinz, 47447 Moers (DE)
(74) Vertreter: BIP Patents
(86) Internationale Anmeldenummer: PCT/EP2011/062832
(87) Internationale Veröffentlichungsnummer: WO 2012/013677

(56) Entgegenhaltungen:
- EP-A1- 0 212 602
- EP-A2- 0 944 578
- US-A- 3 684 740

## Beschreibung

Die vorliegende Erfindung betrifft ein Verfahren zur Wiederherstellung der Aktivität von gebrauchten Katalysatoren für die Hydrierung aromatischer Nitroverbindungen, bei dem in periodischen Abständen eine Regenerierung umfassend mindestens eine erste Abbrennstufe, eine erste Waschstufe, eine zweite Abbrennstufe und eine zweite Waschstufe durchgeführt wird.

Ein Reaktivierungsverfahren für auf *α*-Aluminiumoxid geträgerte Edelmetallkatalysatoren wird in US 3,684,740 (äquivalent zu DE OS 20 28 202**)** beschrieben. Diesem Reinigungsverfahren für Katalysatoren liegt zu Grunde, dass die Katalysator-Aktivität und die Katalysator-Selektivität durch eine Regenerierung umfassend das Abbrennen kohlenstoffhaltiger Ablagerungen und anschließende einmalige Wäsche mit Wasser wieder hergestellt werden kann. Diese Druckschrift offenbart, dass beim fortwährenden Betrieb einer katalytischen Hydriereinheit die Aktivität und Selektivität des Katalysators wegen der Abscheidung von Polymerspezies und möglichen anderen Giften auf dem Katalysator immer weiter abnimmt. Dies macht eine periodische Regenerierung notwendig. Die Standard-Regenerierung nach dem Stand der Technik zum Zeitpunkt der Anmeldung von US 3,684,740 umfasste meist nur das Abbrennen von kohlenstoffhaltigem Material, indem man ein Inertgas, das geringe Mengen Sauerstoff enthält, bei erhöhten Temperaturen auf den Katalysator einwirken ließ. Nach einigen nur aus einem Abbrennschritt bestehenden Regenerationen kann ein Katalysator jedoch jegliche Hydrieraktivität verlieren. US 3,684,740 lehrt nun, dass ein Waschen des Katalysators mit Wasser, nach der Abbrennstufe vorgenommen, die Katalysator-Aktivität und -Selektivität über zusätzliche Zeiträume hinweg auf eine bedeutend höhere Stufe, ja sogar nahezu auf anfängliche Höhen, zurückführen kann. Das Waschen des Katalysators erfolgt dabei kontinuierlich durch Durchleiten von Wasser durch ein fixiertes Katalysatorbett. Laut US 3,684,740 ergeben sich dadurch beträchtliche Einsparungen an Katalysatorkosten und Ausfallzeiten des Reaktors.

Bei lediglich einmaligem Abbrennen kohlenstoffhaltiger Ablagerungen und anschließender Wasserwäsche kann im Falle von gebrauchtem, auf Aluminiumoxid geträgertem Katalysator enthaltend Palladium, Vanadium und Blei, wie er zur Hydrierung von Nitroaromaten verwendet wird (siehe bspw. EP 0 944 578 A2**,** EP 0 011 090 A1**,** EP 0 696 574 B1**,** EP 0 696 573 B1**,** EP 1 882 681 A1**),** in der Praxis die ursprüngliche Katalysator-Aktivität jedoch oft nicht wieder hergestellt werden. Dies ist Verunreinigungen des Katalysators geschuldet, die z. B. durch salzhaltige Nitroaromaten (siehe EP 1 816117 B1 für Details dieser Problematik), Korrosion von Anlagenteilen oder aber verunreinigten Wasserstoff auftreten, und die sich durch die Verfahren des Standes der Technik nicht entfernen lassen. Wird ein nach dem Stand der Technik gereinigter Katalysator bei Temperaturen von 270 °C bis 280 °C erneut mit Luft behandelt, so wird der Abbrand weiteren kohlenstoff-haltigen Materials beobachtet, was die Unvollständigkeit der Reinigungsprozedur anzeigt. Dies führt zu immer schlechteren Betriebszeiten von Anlagen für die Nitroaromatenhydrierung und damit zur Notwendigkeit, den gebrauchten Katalysator durch neuen ersetzen zu müssen, was sehr kostspielig ist.

Eine Aufgabe der vorliegenden Erfindung war es daher, ein Verfahren zur Wiederherstellung der Aktivität von in der Hydrierung von Nitroaromaten zu aromatischen Aminen eingesetzten Katalysatoren bereitzustellen, das es ermöglicht, Katalysator über lange Zeiträume hinweg immer wieder einsetzen zu können, sodass der Neukauf von Katalysator auf ein Minimum reduziert wird.

Die Aufgabe wurde gelöst durch ein Verfahren zur Wiederherstellung der Aktivität eines in der Hydrierung von Nitroaromaten eingesetzten Katalysators durch Regeneration in periodischen Abständen, wobei die Regeneration des Katalysators mindestens die folgenden Stufen umfasst:
(i) eine erste Abbrennstufe umfassend eine Behandlung des Katalysators mit Regeneriergas enthaltend Sauerstoff im Bereich von 0,1 Vol.% bis 90 Vol.-%, bevorzugt von 5 Vol.% bis 50 Vol.-%, besonders bevorzugt von 15 Vol.% bis 25 Vol.-%, bezogen auf das Gesamtvolumen des Regeneriergases;
(ii) eine erste Waschstufe umfassend eine diskontinuierliche Behandlung des Katalysators aus (i) mit Wasser unter mechanischer Durchmischung im Volumenverhältnis Katalysator : Wasser im Bereich von 1 : 1 bis 1 : 100, bevorzugt im Bereich von 1 : 2 bis 1 : 75, besonders bevorzugt im Bereich von 1 : 4 bis 1 : 50, ganz besonders bevorzugt im Bereich von 1 : 5 bis 1 : 25;
(iii) eine zweite Abbrennstufe umfassend eine Behandlung des Katalysators mit Regeneriergas enthaltend Sauerstoff im Bereich von 0,1 Vol.% bis 90 Vol.-%, bevorzugt von 5 Vol.-% bis 50 Vol.-%, besonders bevorzugt von 15 Vol.-% bis 25 Vol.-%, bezogen auf das Gesamtvolumen des Regeneriergases;
(iv) eine zweite Waschstufe umfassend eine diskontinuierliche Behandlung des Katalysators aus (iii) mit Wasser unter mechanischer Durchmischung im Volumenverhältnis Katalysator : Wasser im Bereich von 1 : 1 bis 1 : 100, bevorzugt im Bereich von 1 : 2 bis 1 : 75, besonders bevorzugt im Bereich von 1 : 4 bis 1 : 50, ganz besonders bevorzugt im Bereich von 1 : 5 bis 1 : 25.

An diese mindestens vier Reinigungsstufen können sich gegebenenfalls weitere Abbrenn- und Waschstufen wie (i) bzw. (iii) und (ii) bzw. (iv) anschließen. Die letzte Reinigungsstufe kann dabei sowohl eine Wasch- als auch eine Abbrennstufe sein.

Bevorzugte aromatische Amine, in deren Herstellung der erfindungsgemäß regenerierte Katalysator zum Einsatz kommt, sind Verbindungen der Formel 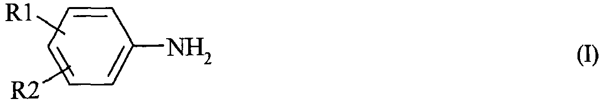 in der R1 und R2 unabhängig voneinander Wasserstoff, Methyl oder Ethyl bedeuten, wobei R1 zusätzlich NH₂ bedeuten kann. Diese werden durch Hydrierung, bevorzugt Gasphasenhydrierung, von Nitroaromaten der Formel 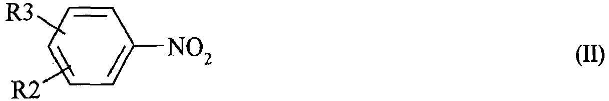 in der R2 und R3 unabhängig voneinander Wasserstoff, Methyl oder Ethyl bedeuten, wobei R3 zusätzlich NO₂ bedeuten kann, erhalten. Besonders bevorzugte aromatische Amine sind Anilin (R1 = R2 = H) und Toluylendiamin (R1 = NH₂; R2 = Methyl), hergestellt aus Nitrobenzol (R2 = R3 = H) bzw. Dinitrotoluol (R2 = Methyl; R3 = NO₂). Ganz besonders bevorzugtes aromatisches Amin ist Anilin. Die Erfindung betrifft demnach insbesondere auch die Verwendung eines Katalysators, dessen Aktivität in periodischen Abständen durch die erfindungsgemäße Regeneration umfassend mindestens die Stufen (i) bis (iv) wiederhergestellt wird, in der Hydrierung von Nitrobenzol oder Dinitrotoluol zu den entsprechenden Aminen.

Die Hydrierung der Nitroverbindungen erfolgt bevorzugt kontinuierlich und unter Rückführung nicht umgesetzten Wasserstoffs in die Reaktion. Bevorzugt ist der Katalysator in Form fester Katalysatorbetten angeordnet. Besonders bevorzugt sind Reaktionsführungen wie in EP 0 944 578 A2 (isotherme Fahrweise) und in EP 0 696 574 B1, EP 0 696 573 B1, EP 1 882 681 A1 (adiabate Fahrweise) beschrieben. Ganz besonders bevorzugt ist die in EP 1 882 681 A1 beschriebene Fahrweise.

Im Laufe eines Betriebszyklus verliert der Katalysator zunehmend an Aktivität als Folge von Verkokungen und Ablagerung von Salzen (z. B stammend aus Verunreinigungen im Nitroaromaten). Unter "Aktivität des Katalysators" wird im Rahmen dieser Erfindung die Fähigkeit des Katalysators verstanden, den eingesetzten Nitroaromaten möglichst lange und möglichst vollständig umzusetzen. Die Aktivität kann dabei auf verschiedene Weisen quantifiziert werden; bevorzugt geschieht dies über die Dauer eines Betriebszyklus, welche auch als "Standzeit" bezeichnet wird. Unter Standzeit wird der Zeitraum zwischen Beginn der Hydrierung und dem Auftreten signifikanter Mengen an nicht umgesetzten Nitroaromaten im rohen Reaktionsprodukt, welches das Beenden der Reaktion erforderlich macht, verstanden. "Signifikante Mengen" sind dabei Massenanteile an Nitroaromaten von größer als 1000 ppm, bevorzugt größer als 500 ppm, besonders bevorzugt größer als 100 ppm, bezogen auf die Gesamtmasse des organischen Anteils des rohen Reaktionsproduktes.

Ist ein solcher signifikanter Gehalt an Nitroaromat im Reaktionsprodukt erreicht, wird die Hydrierung unterbrochen und der Katalysator regeneriert. Je nachdem, wie groß die erreichte Standzeit im Vergleich zur Standzeit, die mit einem frischen Katalysator desselben Katalysatorsystems erzielt werden kann ("ideale Standzeit"), ausfällt, werden verschiedene Methoden angewandt, um den Katalysator zu regenerieren, d. h. seine Aktivität möglichst vollständig wiederherzustellen. Unter einem Katalysatorsystem wird hier ein bestimmter Typ von Katalysator verstanden, also bspw. der in EP 0 011 090 A1 beschriebene Katalysatortyp bestehend aus Pd (9 g pro Liter Träger), V (9 g pro Liter Träger) und Blei (3 g pro Liter Träger) auf α-Aluminiumoxid.

Liegt die erzielte Standzeit nur wenig unter der idealen Standzeit, reicht im Allgemeinen eine einfache Abbrennstufe zur Entfernung von kohlenstoffhaltigen Ablagerungen, um die Aktivität des Katalysators in ausreichendem Maße wiederherzustellen. Ist jedoch die erzielte Standzeit deutlich geringer als die ideale Standzeit, so wird bevorzugt das erfindungsgemäße Regenerationsverfahren mindestens umfassend die Stufen (i) bis (iv) durchgeführt. Bevorzugt wird die erfindungsgemäße Regeneration mindestens umfassend die Stufen (i) bis (iv) immer dann durchgeführt, wenn die Aktivität des in der Hydrierung eingesetzten Katalysators unter 30 %, bevorzugt unter 50 %, besonders bevorzugt unter 80 % der Aktivität eines frischen Katalysators desselben Katalysatorsystems fällt, wobei die Aktivität bevorzugt über das Verhältnis von erzielter Standzeit zu idealer Standzeit quantifiziert wird. Das erfindungsgemäße Regenerationsverfahren wird also bevorzugt in "periodischen Abständen" durchgeführt, welche sich über den festgestellten Aktivitätsverlust des Katalysators definieren.

Die Abbrennstufen (i) und (iii) sowie gegebenenfalls weitere Abbrennstufen des erfindungsgemäßen Verfahrens werden bevorzugt bei Temperaturen zwischen 200 °C und 500 °C, bevorzugt zwischen 240 °C und 400 °C, besonders bevorzugt zwischen 260 °C und 350 °C, ganz besonders bevorzugt zwischen 270 °C und 300 °C durchgeführt, wobei als Regeneriergas enthaltend Sauerstoff bevorzugt Luft eingesetzt wird. Zu Beginn einer Abrennstufe wird die Luft im Allgemeinen noch mit Stickstoff verdünnt, um eine zu starke Temperaturerhöhung zu vermeiden. Mindestens die Abbrennstufe (i) wird bevorzugt im Reaktor für die Hydrierung durchgeführt.

Die Waschstufen (ii) und (iv) sowie gegebenenfalls weitere Waschstufen erfolgen diskontinuierlich unter mechanischer Durchmischung, d. h. der zu reinigende Katalysator wird in einem geeigneten Apparat mit Wasser im oben genannten Volumenverhältnis bedeckt und die erhaltene Suspension mechanisch durchmischt. Im Fall des Katalysators ist das in die Berechnung des Volumenverhältnisses eingehende Katalysatorvolumen das Schüttvolumen. Die mechanische Durchmischung in den Waschstufen (ii) und (iv) sowie gegebenenfalls in weiteren Waschstufen
erfolgt bevorzugt durch Rühren, wobei die Rührenergie so eingestellt wird, dass 99,0 Massen-% bis 100 Massen-%, bevorzugt 99,5 Massen-% bis 100 Massen-%, besonders bevorzugt 99,9 Massen-% bis 100 Massen-% des Katalysators, bezogen auf die Gesamtmasse des in der jeweiligen Stufe zu waschenden Katalysators, strukturell intakt bleiben;
und
wird bevorzugt für Zeiträume zwischen 2 Minuten und 60 Minuten, besonders bevorzugt für Zeiträume zwischen 10 Minuten und 30 Minuten, durchgeführt.

Hierdurch wird ein besonders intensiver Kontakt zwischen dem zu reinigenden Katalysator und dem Waschwasser hergestellt, der eine effektivere Reinigung als mit dem kontinuierlichen Waschverfahren aus US 3,684,740 ermöglicht. "Strukturell intakt" bedeutet dabei, dass die Partikel aus denen der Katalysator besteht (z. B. Kugeln oder andere Formkörper), in der Waschprozedur nicht zerbrechen. Es kann höchstens oberflächlicher Abrieb auftreten.

Neben dem Rühren kommen noch andere Methoden der mechanischen Durchmischung in Frage, beispielsweise durch gezieltes Erzeugen von Strömungen, z. B. durch Einleitung von Gasen wie bspw. Stickstoff oder Luft oder durch Umpumpen des Waschmediums. Als Apparate für die Durchführung der Waschstufen (ii) und (iv) sowie gegebenenfalls weiterer Waschstufen eignen sich beispielsweise Waschbütten oder Betonmischer. Sind geeignete Vorrichtungen für die mechanische Durchmischung im Reaktor vorhanden, kann auch der Reaktor selbst eine geeignete Waschvorrichtung sein. In dem Falle erübrigt sich die Entnahme und Wiedereinführung des Katalysators aus dem bzw. in den Reaktor.

Bevorzugt hat das Wasser in den Waschstufen (ii) und (iv) sowie gegebenenfalls weiteren Waschstufen eine Temperatur zwischen 4 °C und 100 °C, besonders bevorzugt zwischen 10 °C und 70 °C und ganz bevorzugt zwischen 15 °C und 50 °C. Das zum Waschen eingesetzte Wasser muss weitgehend bis vollständig frei von die Katalysator-Aktivität beeinträchtigenden Ionen (z. B. Sulfat) sein. Daher werden die besten Resultate mit destilliertem Wasser erzielt. Besonders wirtschaftlich ist es, wenn die aromatischen Amine durch Gasphasenhydrierung der korrespondierenden Nitroaromaten hergestellt werden, das so jeweils erhaltene gasförmige, rohes Amin und Reaktionswasser enthaltende Reaktionsprodukt kondensiert und durch Phasentrennung in eine organische und eine wässrige Phase getrennt wird, und das Wasser für die Waschstufe (ii) und / oder die Waschstufe (iv) und / oder weitere Waschstufen aus der so gewonnenen wässrigen Phase stammt. In dieser Ausführungsform stammt also das Wasser für die Waschstufe (ii) und / oder die Waschstufe (iv) aus der wässrigen Phase, welche durch Phasentrennung des kondensierten rohen Reaktionsprodukts einer Gasphasenhydrierung von Nitroaromaten erhalten wurde. Vor Durchführung der Waschstufen (ii) und (iv) und gegebenenfalls weiterer Waschstufen lässt man den mit Regeneriergas behandelten Katalysator abkühlen, und zwar bevorzugt auf die Temperatur des Waschwassers.

Für die auf die erste Wäsche folgenden Waschstufen kann die Waschflüssigkeit aus den vorhergehenden Waschstufen wiederverwendet werden, sofern deren Belastung mit die Katalysator-Aktivität beeinträchtigenden Ionen (z. B. Sulfat) nicht zu groß ist. Gegebenenfalls kann zwischendurch eine Aufarbeitung dieses Waschwassers, z. B. durch Filtration oder Sedimentation und Abdekantieren, erfolgen. Bevorzugt wird die letzte Waschstufe mit neuem Waschwasser durchgeführt, besonders bevorzugt mit Wasser aus der wie oben beschrieben in der Phasentrennung erhaltenen wässrigen Phase.

Nach Beendigung der diskontinuierlichen Behandlungen des Katalysators mit Wasser wird das Waschwasser entfernt, bevorzugt durch Filtration. Im Anschluss daran wird der Katalysator bevorzugt durch Abspülen mit fließendem Wasser von oberflächlich anhaftenden Resten an Verunreinigungen befreit.

Der feuchte Katalysator wird vor der folgenden Abbrennstufe bzw. vor dem erneuten Einsatz in der Hydrierung bevorzugt getrocknet. Bevorzugt erfolgt die Trocknung im warmen Luftstrom, gegebenenfalls bei vermindertem Druck, bevorzugt im Bereich von 50 mbar bis 1000 mbar.

Der gebrauchte Katalysator wird während der Regenerierung mindestens umfassend die Stufen (i) bis (iv) bevorzugt mindestens einmal gesiebt, um Staubpartikel abzutrennen. Bevorzugt geschieht dies zwischen den Abbrennstufen und den Waschstufen. Besonders bevorzugt wird der zu regenerierende Katalysator vor Stufe (ii) und / oder vor Stufe (iv) und / oder vor weiteren Waschstufen zur Abtrennung von Staubpartikeln mit einem mittleren Partikeldurchmesser < 1 mm gesiebt. Das Sieben des Katalysators erfolgt mittels dem Fachmann bekannten Apparaturen und Methoden. Es kommen z. B. Zyklone, Siebe etc. zum Einsatz. Durch diesen zusätzlichen Reinigungsschritt lässt sich die Belastung des Abwassers reduzieren und so die Entsorgung vereinfachen.

Das erfindungsgemäße Regenerationsverfahren eignet sich besonders für die Reinigung der in EP 0 944 578 A2, EP 0 011 090 A1, EP 0 696 574 B1, EP 0 696 573 B1 und EP 1 882 681 A1 beschriebenen Hydrier-Katalysatoren. Insbesondere eignet es sich für die Wiederherstellung der Aktivität eines Katalysators für die Hydrierung aromatischer Nitroverbindungen, bei dem der Katalysator katalytisch aktive Komponenten auf einem Aluminiumoxid-Träger mit einem mittleren Durchmesser der Aluminiumoxid-Partikel zwischen 1,0 mm und 7,0 mm und einer BET-Oberfläche von weniger als 20 m²/g enthält, und bei dem die aktiven Komponenten mindestens umfassen:
(a) 1 - 100 g/l_{Träger} mindestens eines Metalls der Gruppen 8 bis 12 des Periodensystems der Elemente, und
(b) 0 - 100 g/l_{Träger} mindestens eines Übergangsmetalls der Gruppen 4 bis 6 und 12 des Periodensystems der Elemente, sowie
(c) 0 - 100 g/l_{Träger} mindestens eines Metalls der Hauptgruppenelemente der Gruppen 14 und 15 des Periodensystems der Elemente.

(Die Gruppen des Periodensystems der Elemente werden in dieser Druckschrift gemäß der IUPAC-Empfehlung von 1986 gezählt.)

Der Aluminiumoxidträger hat bevorzugt annähernd Kugelform und bevorzugt einen Durchmesser im Bereich von 1,0 mm bis 7,0 mm

Die Bruchhärte von 20-mal gewaschenem und getrocknetem gebrauchtem Katalysator des Katalysatortyps Pd (9 g/l_{Träger}) / V (9 g/l_{Träger}) / Pb (3 g/l_{Träger}) auf α-Aluminiumoxid bleibt unverändert, das heißt, der Katalysator wird durch vielmaliges Waschen nicht in seiner mechanischen Stabilität beeinträchtigt.

### Beispiele:

Als Versuchsanlage für die Beispielreaktionen dient ein 500 mm langes Reaktionsrohr aus Edelstahl, das über einen Verdampfer mit Edukten beschickt wird. Mittels Dosierpumpen wird Nitrobenzol von oben in den Verdampfer gepumpt. Der Wasserstoff wird von unten in den Verdampfer geleitet, der durch Ölthermostaten beheizt wird (ca. 250 °C), sodass das von oben eingepumpte Nitrobenzol im Gegenstrom verdampfen kann. Die Wasserstoff-Versorgung wird vor dem Verdampfer durch Massedurchflussregler geregelt. Die Belastung wurde in allen Beispielversuchen auf 1 g_{Nitroaromat}/(ml_{Katalysator} · h) und das Wasserstoff : Nitrobenzol-Verhältnis auf ca. 80 : 1 festgelegt.

Auf einem Sieb innerhalb des Reaktionsrohrs wird eine 400 mm hohe Schüttung des Katalysators platziert. Nach Austritt des Reaktors wird das Reaktionsprodukt mit Wasser gekühlt. Die nicht flüchtigen Bestandteile werden so auskondensiert und in einem nachgeschalteten Abscheider von den gasförmigen Komponenten getrennt. Die flüssigen Bestandteile werden aus dem Abscheider in den Produktsammelbehälter geführt und dort aufgefangen (Glasbehälter). Vor dem Sammelbehälter befindet sich eine Probennahmestelle, an der in regelmäßigen Zeitabständen Proben des Produkts gezogen werden können. Diese werden gaschromatographisch analysiert. Die Standzeit des Katalysators entspricht der Zeit vom Beginn der Reaktion bis kein vollständiger Umsatz des Nitrobenzols mehr erreicht wird und an der Probennahmestelle > 0,1 % Nitrobenzol im Produkt mittels Gaschromatographie detektiert werden.

Alle Beispiele wurden mit dem Katalysatorsystem 9 g/ l-_{Träger} Pd, 9 g/ l_{Träger} V, 3 g/ l_{Träger} Pb auf α-Aluminiumoxid durchgeführt (siehe EP 0 011 090 A1). Es wurden unterschiedlich gealterte und vorbehandelte Katalysatoren dieses Katalysatorsystems eingesetzt; die Reaktionsversuche wurden jeweils abgebrochen, wenn die Standzeit des Katalysators erreicht war.

### Beispiel 1: Vergleichsbeispiel: "Frischer Katalysator"

Frisch hergestellter Katalysator wird im Reaktionsrohr platziert und zunächst mit Stickstoff, dann mit Wasserstoff gespült. Im Anschluss wurde der Katalysator über einen Zeitraum von 48 h bei 240 °C mit 1000 l/h Wasserstoff beaufschlagt. Dann wurde die Nitrobenzolbelastung langsam auf den gewünschten Wert von 1 g_{Nitroaromat}/(ml_{Katalysator} · h) erhöht, sodass die Temperatur im Reaktor nicht über 450 °C anstieg, und die Wasserstoffzugabe wurde so eingestellt, dass das molare Verhältnis von Wasserstoff : Nitrobenzol 80 : 1 betrug.

### Beispiel 2: Vergleichsbeispiel: "regeneriert ohne Wäsche"

Ein gebrauchter Katalysator mit geringer Restaktivität, der zur Hydrierung von Nitrobenzol zu Anilin verwendet wurde, wurde durch eine Abbrennstufe regeneriert. Dazu wurde er zunächst auf 270 °C erwärmt und dann mit Luftstrom beaufschlagt, um Verkokungen abzubrennen. Dies wurde so lange durchgeführt, bis keine Wärmeabgabe mehr zu erkennen war und der CO₂-Gehalt im Abgasstrom auf weniger als 0,2 % gefallen war (bestimmt durch IR-Photometrie). Danach wurde das System mit Stickstoff inertisiert und der Katalysator über einen Zeitraum von 48 h bei 240 °C mit 1000 l/h Wasserstoff beaufschlagt. Die Nitrobenzolbelastung wurde dann langsam auf den gewünschten Wert von 1 g_{Nitroaromat}/(ml_{Katalysator}· h) erhöht, sodass die Temperatur im Reaktor nicht über 450 °C anstieg, und die Wasserstoffzugabe wurde so eingestellt, dass das molare Verhältnis von Wasserstoff : Nitrobenzol 80 : 1 betrug.

### Beispiel 3: Vergleichsbeispiel: "reaktiviert gemäß DE-OS-20 28 202"

Eingesetzt wurde gebrauchter Katalysator mit geringer Restaktivität aus der gleichen Charge wie in Beispiel 2, der diesmal in Anlehnung an DE-OS-20 28 202 reaktiviert wurde, d. h. er wurde zunächst auf 270 °C erwärmt und dann mit Luftstrom beaufschlagt, um Verkokungen abzubrennen. Dies wurde so lange durchgeführt, bis keine Wärmeabgabe mehr zu erkennen war und der CO₂-Gehalt im Abgasstrom auf weniger als 0,2 % gefallen war. Nach dem Abkühlen wurde der Katalysator mit destilliertem Wasser kontinuierlich gewaschen, bis der vom Katalysator ablaufende Waschwasserstrom für ca. 20 Minuten lang klar war und dann mit heißem Inertgas getrocknet.

Zur Inertisierung wurde der Katalysator zunächst mit Stickstoff gespült. Anschließend wurde der Katalysator über einen Zeitraum von 48 h bei 240 °C mit 1000 l/h Wasserstoff beaufschlagt. Dann wurde die Nitrobenzolbelastung langsam auf den gewünschten Wert von 1 g_{Nitroaromat}/(ml_{Katalysator} · h) erhöht, sodass die Temperatur im Reaktor nicht über 450 °C anstieg, und die Wasserstoffzugabe wurde so eingestellt, dass das molare Verhältnis von Wasserstoff : Nitrobenzol 80 : 1 betrug.

### Beispiel 4: Vergleichsbeispiel: erneutes Abbrennen nach der Wasserwäsche

Ein Teil der in Beispiel 3 hergestellten Katalysatorprobe wurde nach der Reaktivierungsprozedur nicht wie üblich inertisiert und zur Reaktion verwendet, sondern zunächst noch einmal auf 270 °C erwärmt und mit Luft überströmt. Dabei konnten in der Abluft erneut CO₂-Gehalte von mehr als 1,5 % sowie ein Temperaturanstieg von mehreren Grad beobachtet werden, was auf ein weiteres Abbrennen von kohlenstoffhaltigem Material hindeutet. Es wurde wieder so lange Luft über den Katalysator geleitet, bis der CO₂-Gehalt in der Abluft unter 0,2 % fiel. Danach wurde Das System mit Stickstoff inertisiert, und der Katalysator über einen Zeitraum von 48 h bei 240 °C mit 1000 l/h Wasserstoff beaufschlagt. Die Nitrobenzolbelastung wurde anschließend langsam auf den gewünschten Wert von 1 g_{Nitroaromat}/(ml_{Katalysator} · h) erhöht, so dass die Temperatur im Reaktor nicht über 450 °C anstieg und die Wasserstoffzugabe wurde so eingestellt, dass das molare Verhältnis von Wasserstoff: Nitrobenzol 80 : 1 betrug.

### Beispiel 5: erfindungsgemäß: "mehrfache Reaktivierung"

Der gebrauchte Katalysator, der auch als Ausgangsmaterial für die Behandlung in Beispiel 2 diente, wurde zunächst wie üblich im Luftstrom bei 270 °C regeneriert, bis keine Wärmeabgabe mehr erfolgte und der CO₂-Gehalt im Abgas unter 0,2 % gefallen war. Anschließend wurde der Katalysator in einer Mischapparatur mit dem 5-fachen Volumen Wasser beaufschlagt und für 10 Minuten bei Raumtemperatur gerührt. Der so gewaschene Katalysator wurde unter einer Waschbrause mit Wasser abgespült und bei 100 °C getrocknet. Diese Prozedur aus Abbrennen im Luftstrom und Waschen mit Wasser wurde noch zwei weitere Male durchgerührt, bevor der Katalysator im Reaktor inertisiert und über einen Zeitraum von 48 h bei 240 °C mit 1000 l/h Wasserstoff beaufschlagt wurde. Dann wurde die Nitrobenzolbelastung langsam auf den gewünschten Wert von 1 g_{Nitroaromat}/ml_{Katalysator} · h) erhöht, sodass die Temperatur im Reaktor nicht über 450 °C anstieg, und die Wasserstoffzugabe wurde so eingestellt, dass das molare Verhältnis von Wasserstoff: Nitrobenzol 80 : 1 betrug.

### Beispiel 6: erfindungsgemäß: "mehrfache Reaktivierung mit Sieben"

Der gebrauchte Katalysator, der auch als Ausgangsmaterial für die Behandlung in Beispiel 2 diente wurde zunächst wie üblich im Luftstrom bei 270 °C regeneriert, bis keine Wärmeabgabe mehr erfolgte und der CO₂-Gehalt im Abgas unter 0,2 % gefallen war. Der Katalysator wurde dem Reaktor entnommen und vorhandene Feinanteile (Abrieb, staubartige Verunreinigungen) wurden durch Sieben des Katalysators mit einem Sieb der Porengröße 1 mm entfernt. Anschließend wurde der Katalysator in einer Mischapparatur mit dem 5-fachen Volumen Wasser beaufschlagt und für 10 Minuten gerührt. Der so gewaschene Katalysator wurde unter einer Waschbrause mit Wasser abgespült und bei 100 °C getrocknet. Diese Prozedur aus Abbrennen im Luftstrom und Waschen mit Wasser wurde noch zwei weitere Male durchgeführt, bevor der Katalysator im Reaktor inertisiert und über einen Zeitraum von 48 h bei 240 °C mit 1000 l/h Wasserstoff beaufschlagt wurde. Dann wurde die Nitrobenzolbelastung langsam auf den gewünschten Wert von 1 g_{Nitroaromat}/(ml_{Katalysator} · h) erhöht, sodass die Temperatur im Reaktor nicht über 450 °C anstieg, und die Wasserstoffzugabe wurde so eingestellt, dass das molare Verhältnis von Wasserstoff : Nitrobenzol 80 : 1 betrug.

Die folgende Tabelle fasst die Resultate zusammen:

**Tabelle 1: Ergebnisse der Beispiele**

| Nr. | Art | Standzeit | Na-Gehalt des Katalysators^{[a]} |
|---|---|---|---|
| Beispiel 1 | Vergleich (frischer Kat.) | 987 h | 56 ppm |
| Beispiel 2 | Vergleich | 152 h | 0,18 % |
| Beispiel 3 | Vergleich | 318 h | 0,12 % |
| Beispiel 4 | Vergleich | 376 h | 0,11 % |
| Beispiel 5 | erfindungsgemäß | 826 h | 370 ppm |
| Beispiel 6 | erfindungsgemäß | 818 h | 355 ppm |

| | | | |
|---|---|---|---|
| [a] Angegeben sind Massenanteile, bestimmt durch ICP-OES. | | | |

Wie man sieht, ist die mit dem nur durch Abbrennen regenerierten Katalysator erzielte Standzeit (Beispiel 2) erheblich geringer als die mit frischem Katalysator erzielte (Beispiel 1). Schließt sich an die Abbrennstufe noch eine Waschstufe an (Beispiel 3), steigt die Standzeit zwar wieder, jedoch nur um ein Drittel von 150 h auf 200 h. Weiteres Abbrennen führt dann zwar noch einmal zu einem spürbaren Anstieg der Standzeit von 200 h auf 300 h (Beispiel 4), jedoch werden nur mit der erfindungsgemäßen Vorgehensweise Standzeiten erzielt, die wieder in der gleichen Größenordnung wie die ideale Standzeit liegen.

Der Natriumgehalt ist ein wichtiges Indiz für die Verunreinigung des Katalysators und liegt bei den erfindungsgemäßen Beispielen deutlich niedriger als bei den Vergleichsbeispielen.

## Patentansprüche

1. Verfahren zur Wiederherstellung der Aktivität eines in der Hydrierung von Nitroaromaten eingesetzten Katalysators durch Regeneration in periodischen Abständen, **dadurch gekennzeichnet, dass**
die Regeneration des Katalysators mindestens die folgenden Stufen umfasst:
(i) eine erste Abbrennstufe umfassend eine Behandlung des Katalysators mit Regeneriergas enthaltend Sauerstoff im Bereich von 0,1 Vol.-% bis 90 Vol.-%, bezogen auf das Gesamtvolumen des Regeneriergases;
(ii) eine erste Waschstufe umfassend eine diskontinuierliche Behandlung des Katalysators aus (i) mit Wasser unter mechanischer Durchmischung im Volumenverhältnis Katalysator: Wasser im Bereich von 1 : 1 bis 1 : 100;
(iii) eine zweite Abbrennstufe umfassend eine Behandlung des Katalysators aus (ii) mit Regeneriergas enthaltend Sauerstoff im Bereich von 0,1 Vol.-% bis 90 Vol.-%, bezogen auf das Gesamtvolumen des Regeneriergases;
(iv) eine zweite Waschstufe umfassend eine diskontinuierliche Behandlung des Katalysators aus (iii) mit Wasser unter mechanischer Durchmischung im Volumenverhältnis Katalysator: Wasser im Bereich von 1 : 1 bis 1 : 100.

2. Verfahren nach Anspruch 1, bei dem das Regeneriergas in den Abrennstufen (i) und (iii) Sauerstoff jeweils im Bereich von 5 Vol.% bis 50 Vol.-%, bezogen auf das Gesamtvolumen des Regeneriergases, enthält und das Volumenverhältnis von Katalysator : Wasser in den Waschstufen (ii) und (iv) jeweils im Bereich von 1 : 2 bis 1 : 75 liegt.

3. Verfahren nach Anspruch 1, bei dem das Regeneriergas in den Abrennstufen (i) und (iii) Sauerstoff jeweils im Bereich von 15 Vol.-% bis 25 Vol.-%, bezogen auf das Gesamtvolumen des Regeneriergases, enthält und das Volumenverhältnis von Katalysator: Wasser in den Waschstufen (ii) und (iv) jeweils im Bereich von 1 : 5 bis 1 : 25 liegt.

4. Verfahren nach Anspruch 1, bei dem der Katalysator katalytisch aktive Komponenten auf einem Aluminiumoxid-Träger mit einem mittleren Durchmesser der Aluminiumoxid-Partikel zwischen 1,0 mm und 7,0 mm und einer BET-Oberfläche von weniger als 20 m²/g enthält, und bei dem die aktiven Komponenten mindestens umfassen:
(a) 1 - 100 g/l_{Träger} mindestens eines Metalls der Gruppen 8 bis 12 des Periodensystems der Elemente, und
(b) 0 - 100 g/l_{Träger} mindestens eines Übergangsmetalls der Gruppen 4 bis 6 und 12 des Periodensystems der Elemente, sowie
(c) 0 - 100 g/l_{Träger} mindestens eines Metalls der Hauptgruppenelemente der Gruppen 14 und 15 des Periodensystems der Elemente.

5. Verfahren nach Anspruch 1, bei dem der zu regenerierende Katalysator vor Stufe (ii) und / oder vor Stufe (iv) zur Abtrennung von Staubpartikeln mit einem mittleren Partikeldurchmesser < 1 mm gesiebt wird.

6. Verfahren nach Anspruch 1, bei dem das Wasser in den Waschstufen (ii) und (iv) eine Temperatur zwischen 4 °C und 100 °C hat.

7. Verfahren nach Anspruch 1, bei dem die mechanische Durchmischung in den Waschstufen (ii) und (iv)
durch Rühren erfolgt, wobei die Rührenergie so eingestellt wird, dass 99,0 Massen-% bis 100 Massen-% des Katalysators, bezogen auf die Gesamtmasse des in der jeweiligen Stufe zu waschenden Katalysators, strukturell intakt bleiben;
und
für Zeiträume zwischen 2 Minuten und 60 Minuten durchgeführt wird.

8. Verfahren nach Anspruch 1, bei dem die Abrennstufen (i) und (iii) bei Temperaturen zwischen 200 °C und 500 °C durchgeführt werden und als Regeneriergas enthaltend Sauerstoff Luft eingesetzt wird.

9. Verfahren nach Anspruch 1, bei dem die Regeneration umfassend die Stufen (i) bis (iv) immer dann durchgeführt wird, wenn die Aktivität des in der Hydrierung eingesetzten Katalysators unter 80 % der Aktivität eines frischen Katalysators desselben Katalysatorsystems fällt.

10. Verfahren nach Anspruch 1, bei dem das Wasser für die Waschstufe (ii) und / oder die Waschstufe (iv) aus der wässrigen Phase, welche durch Phasentrennung des kondensierten rohen Reaktionsprodukts einer Gasphasenhydrierung von Nitroaromaten erhalten wurde, stammt.

11. Verfahren gemäß Anspruch 1, bei dem der Katalysator in der Hydrierung von Nitrobenzol oder Dinitrotoluol eingesetzt wird.

## Claims

1. Process for restoring the activity of a catalyst employed in the hydrogenation of nitroaromatics by regeneration at periodic intervals, **characterized in that**
the regeneration of the catalyst comprises at least the following stages:
(i) a first burning off stage comprising a treatment of the catalyst with regenerating gas containing oxygen in the range of from 0.1 vol.% to 90 vol.%, based on the total volume of the regenerating gas;
(ii) a first washing stage comprising a discontinuous treatment of the catalyst from (i) with water with mechanical mixing, in the volume ratio of catalyst : water in the range of from 1 : 1 to 1 : 100;
(iii) a second burning off stage comprising a treatment of the catalyst from (ii) with regenerating gas containing oxygen in the range of from 0.1 vol.% to 90 vol.%, based on the total volume of the regenerating gas;
(iv) a second washing stage comprising a discontinuous treatment of the catalyst from (iii) with water with mechanical mixing, in the volume ratio of catalyst: water in the range of from 1 : 1 to 1 : 100.

2. Process according to claim 1, in which the regenerating gas burning off stages (i) and (iii) contains oxygen in each case in the range of from 5 vol.% to 50 vol.%, based on the total volume of the regenerating gas, and the volume ratio of catalyst : water in washing stages (ii) and (iv) is in each case in the range of from 1 : 2 to 1 : 75.

3. Process according to claim 1, in which the regenerating gas in burning off stages (i) and (iii) contains oxygen in each case in the range of from 15 vol.% to 25 vol.%, based on the total volume of the regenerating gas, and the volume ratio of catalyst : water in washing stages (ii) and (iv) is in each case in the range of from 1 : 5 to 1 : 25.

4. Process according to claim 1, in which the catalyst contains catalytically active components on an aluminium oxide support with an average diameter of the aluminium oxide particles of between 1.0 mm and 7.0 mm and a BET surface area of less than 20 m²/g, and in which the active components comprise at least:
(a) 1 - 100 g/lₛᵤₚₚₒᵣₜ of at least one metal of groups 8 to 12 of the periodic table of the elements, and
(b) 0 - 100 g/lₛᵤₚₚₒᵣₜ of at least one transition metal of groups 4 to 6 and 12 of the periodic table of the elements, and
(c) 0 - 100 g/lₛᵤₚₚₒᵣₜ of at least one metal of the main group elements of groups 14 and 15 of the periodic table of the elements.

5. Process according to claim 1, in which the catalyst to be regenerated is sieved before stage (ii) and / or before stage (iv) to remove dust particles with an average particle diameter of < 1 mm.

6. Process according to claim 1, in which the water in washing stages (ii) and (iv) has a temperature of between 4 °C and 100 °C.

7. Process according to claim 1, in which the mechanical mixing in washing stages (ii) and (iv)
is carried out by stirring, the stirring energy being adjusted such that 99.0 % by weight to 100 % by weight of the catalyst, based on the total weight of the catalyst to be washed in the particular stage, remains structurally intact;
and
is carried out for periods of time of between 2 minutes and 60 minutes.

8. Process according to claim 1, in which burning off stages (i) and (iii) are carried out at temperatures of between 200 °C and 500 °C and air is employed as the regenerating gas containing oxygen.

9. Process according to claim 1, in which the regeneration comprising stages (i) to (iv) is always carried out when the activity of the catalyst employed in the hydrogenation falls below 80 % of the activity of a fresh catalyst of the same catalyst system.

10. Process according to claim 1, in which the water for washing stage (ii) and / or washing stage (iv) originates from the aqueous phase which was obtained by phase separation of the condensed crude reaction product of a gas phase hydrogenation of nitroaromatics.

11. Process according to claim 1, in which the catalyst is used in the hydrogenation of nitrobenzene or dinitrotoluene.

## Revendications

1. Procédé pour la restauration de l'activité d'un catalyseur utilisé dans l'hydrogénation de composés nitroaromatiques par régénération à des intervalles périodiques, **caractérisé en ce que** la régénération du catalyseur comprend au moins les étapes suivantes :
(i) une première étape de calcination comprenant un traitement du catalyseur par un gaz de régénération contenant de l'oxygène dans la plage de 0,1% en volume à 90% en volume, par rapport au volume total du gaz de régénération ;
(ii) une première étape de lavage comprenant un traitement discontinu du catalyseur de (i) avec de l'eau sous brassage mécanique dans un rapport volumique catalyseur:eau dans la plage de 1:1 à 1:100 ;
(iii) une deuxième étape de calcination comprenant un traitement du catalyseur de (ii) par un gaz de régénération contenant de l'oxygène dans la plage de 0,1% en volume à 90% en volume, par rapport au volume total du gaz de régénération ;
(iv) une deuxième étape de lavage comprenant un traitement discontinu du catalyseur de (iii) avec de l'eau sous brassage mécanique dans un rapport volumique catalyseur:eau dans la plage de 1:1 à 1:100 ;

2. Procédé selon la revendication 1, dans lequel le gaz de régénération dans les étapes de calcination (i) et (iii) contient de l'oxygène à chaque fois dans la plage de 5% en volume à 50% en volume, par rapport au volume total du gaz de régénération et le rapport volumique catalyseur:eau dans les étapes de lavage (ii) et (iv) se situe à chaque fois dans la plage de 1:2 à 1:75.

3. Procédé selon la revendication 1, dans lequel le gaz de régénération dans les étapes de calcination (i) et (iii) contient de l'oxygène à chaque fois dans la plage de 15% en volume à 25% en volume, par rapport au volume total du gaz de régénération et le rapport volumique catalyseur:eau dans les étapes de lavage (ii) et (iv) se situe à chaque fois dans la plage de 1:5 à 1:25.

4. Procédé selon la revendication 1, dans lequel le catalyseur contient des composants catalytiquement actifs sur un support en oxyde d'aluminium présentant un diamètre moyen des particules d'oxyde d'aluminium entre 1,0 mm et 7,0 mm et une surface BET inférieure à 20 m²/g, et dans lequel les composants actifs comprennent au moins :
(a) 1-100 g/lₛᵤₚₚₒᵣₜ d'au moins un métal des groupes 8 à 12 du système périodique des éléments et
(b) 0-100 g/lₛᵤₚₚₒᵣₜ d'au moins un métal de transition des groupes 4 à 6 et 12 du système périodique des éléments ainsi que
(c) 0-100 g/lₛᵤₚₚₒᵣₜ d'au moins un métal des éléments des groupes principaux 14 et 15 du système périodique des éléments.

5. Procédé selon la revendication 1, dans lequel le catalyseur à régénérer est tamisé avant l'étape (ii) et/ou avant l'étape (iv) pour la séparation de particules de poussière présentant un diamètre moyen de particule < 1 mm.

6. Procédé selon la revendication 1, dans lequel l'eau dans les étapes de lavage (ii) et (iv) présente une température entre 4°C et 100°C.

7. Procédé selon la revendication 1, dans lequel le brassage mécanique dans les étapes de lavage (ii) et (iv)
a lieu par agitation, l'énergie d'agitation étant réglée de manière telle que 99,0% en masse à 100% en masse du catalyseur, par rapport à la masse totale du catalyseur à laver dans chaque étape, restent structuralement intacts ; et
est réalisé pendant des laps de temps entre 2 minutes et 60 minutes.

8. Procédé selon la revendication 1, dans lequel les étapes de calcination (i) et (iii) sont réalisées à des températures entre 200°C et 500°C et de l'air est utilisé comme gaz de régénération contenant de l'oxygène.

9. Procédé selon la revendication 1, dans lequel la régénération comprenant les étapes (i) à (iv) est toujours réalisée lorsque l'activité du catalyseur utilisé dans l'hydrogénation chute sous les 80% de l'activité d'un catalyseur frais du même système catalytique.

10. Procédé selon la revendication 1, dans lequel l'eau pour l'étape de lavage (ii) et/ou l'étape de lavage (iv) provient de la phase aqueuse qui a été obtenue par séparation des phases du produit de réaction brut condensé d'une hydrogénation en phase gazeuse de composés nitroaromatiques.

11. océdé selon la revendication 1, dans lequel le catalyseur est utilisé dans l'hydrogénation de nitrobenzène ou de dinitrotoluène.
